# EUROPEAN PATENT APPLICATION

(11) **EP 2 722 033 A1**
(43) Date of publication of application: **23.04.2014**
(21) Application number: 13189159.0
(22) Date of filing: 17.10.2013
(51) Int. Cl.: A61K 9/00, A61K 9/20, A61K 31/4184, A61K 31/4439

(54) **Pharmaceutical Compositions of Dabigatran Free Base**

(30) Priority: 19.10.2012 TR 201212083
(71) Applicant: Sanovel Ilac Sanayi ve Ticaret A.S., 34460 Istanbul (TR)
(72) Inventor: Cifter, Ümit, 34460 Istanbul (TR); Mutlu, Onur, 34460 Istanbul (TR); Hatirnaz, Basak, 34460 Istanbul (TR)
(74) Representative: Sevinç, Erkan

(57) **Abstract**

The present invention relates to a novel pharmaceutical composition comprising dabigatran etexilate free base as an active agent and at least one pharmaceutically acceptable excipient.

## Description

### Technical Field

The present invention relates to a novel pharmaceutical composition comprising dabigatran etexilate free base as an active agent and at least one pharmaceutically acceptable excipient.

### Background of the Invention

Dabigatran etexilate (Formula 1), which is already known from WO 98/37075, is a direct thrombin inhibitor indicated to reduce the risk of stroke and systemic embolism in patients with non-valcular atrial fibrilation.

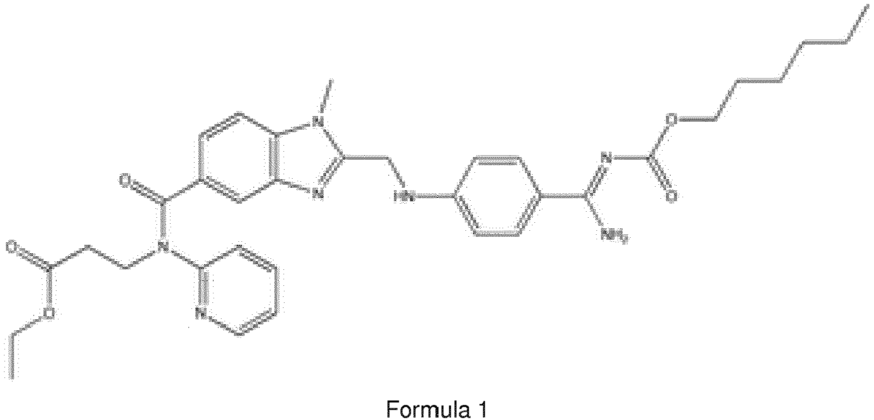

The methane sulphonic acid addition salt of dabigatran etexilate, which is commercially available under the trade name PRADAXA®, is disclosed in EP1870100, wherein also disclosed, pellet composition of dabigatran etexilate methanesulphonate. This composition is formulated with a core material consisting of organic acid and an active layer which encloses the core.

Apart from the methanesulfonate salt of dabigatran etexilate, other acid addition salts of the compound are provided in prior art. For exemple, WO2012/077136 is directed to the oxalate salt of dabigatran etexilate and besides, its hydrochloride salt is identified in EP1877395. These various form of dabigatran etexilate are prepared to facilitate the development of pharmaceutical composition. An active agent should meet some physicochemical requirements in order to be capable of being used in pharmaceutical compositions. These requirements considerably depend on the physicochemical properties of the active agent used in pharmaceutical composition.

These various dabigatran etexilate salts disclosed in prior art, were compared for their physicochemical properties like water solubility, stability, impurity non-hygroscopicity and processability which are important for the development of pharmaceutical compositions. However, there is still a need in the art to develop a pharmaceutical composition comprising a stable, pure, soluble, non-hygroscopic and processable form of dabigatran etexilate.

### Object of the Invention

The main object of the present invention is to use of dabigatran etexilate free base in the preparation of a medicament to reduce the risk of stroke and systemic embolism in patients with non-valcular atrial fibrilation.

Another object of the present invention is to obtain a pharmaceutical composition comprising dabigatran etexilate free base as an active agent and at least one pharmaceutically acceptable excipient, wherein the pharmaceutical composition has advantageous properties by means of improved physicochemical nature of dabigatran etexilate.

Another object of the present invention is to provide a pharmaceutical composition comprising substantially pure dabigatran etexilate free base, wherein the purity degree of dabigatran etexilate free base render more stable the pharmaceutical composition during production and storage.

Another object of the present invention is to provide a stable pharmaceutical composition comprising dabigatran etexilate free base with a reduced moisture content, wherein the content of dabigatran etexilate free base is correctly adjusted by minimizing the absorption of moisture.

Another object of the present invention is to provide a soluble and bioavailable pharmaceutical composition comprising dabigatran etexilate free base and at least one pharmaceutically acceptable excipient.

### Detailed Description of the Invention

The present invention is directed to novel pharmaceutical composition comprising dabigatran etexilate free base as active agent and at least one pharmaceutically acceptable excipient.

As used herein, the term "dabigatran etexilate free base" refers to dabigatran etexilate which is free from other forms of the active moiety, especially acid addition salts.

According to an embodiment of the present invention, said pharmaceutical composition contains dabigatran etexilate free base having an impurity not more than 1%, preferably not more than 0.7% and more preferably not more than 0.4% of the dabigatran etexilate free base content. The impurity of dabigatran etexilate free base may be determined by any methos known in the art, including, but not limited to high performance liquid chromatography (HPLC) analysis.

According to another embodiment of the present invention, said pharmaceutical composition contains dabigatran etexilate free base wherein the moisture content of dabigatran etexilate free base is not more than 1% and preferably not more than 0.5% of dabigatran etexilate free base content.

According to another embodiment of the present invention, said pharmaceutical composition contains dabigatran etexilate free base wherein its solubility in pure water has a value between 0.5 mg/mL and 3 mg/mL, and preferably between 1 mg/mL and 2 mg/mL.

According to prefered embodiment of the present invention, said pharmaceutical composition contains dabigatran etexilate free base having,
i) impurity not more than 1% of dabigatran etexilate free base content,
ii) moisture content not more than 1% of dabigatran etexilate free base content,
iii) solubility in pure water in the range of 0.5 mg/mL and 3 mg/mL.

The dabigatran etexilate free base of the present invention is formed as a solid which is easily handled and particularly well suited to the formulation of pharmaceutical composition. It has been surprisingly discovered that the dabigatran etexilate free base has physicochemical properties (e.g. purity, non-hygroscopicity, solubility) which are more suitable to develop a pharmaceutical composition, than other forms of dabigatran etexilate, e.g. salts. It has also been discovered that pharmaceutical composition formulated with low-hygroscopic and pure dabigatran etexilate free base suitable to the present invention, has improved physicochemical properties such as stability, dissolution, storage and flowability, compared to pharmaceutical compositions described in prior art.

According to prefered embodiment of the present invention, said pharmaceutical composition contains dabigatran etexilate free base having,
i) impurity not more than 0.4% of dabigatran etexilate free base content,
ii) moisture content not more than 0.5% of dabigatran etexilate free base content,
iii) solubility in pure water in the range of 1 mg/mL and 2 mg/mL.

According to an embodiment of the present invention, said pharmaceutical composition contains dabigatran etexilate free base present in an amount from about 5 mg to about 300 mg, and preferably from about 25 mg to about 150 mg. In relative terms, dabigatran etexilate free base may be present in an amount between 5% and 75% by weight of the total composition.

According to an embodiment of the present invention, said pharmaceutical composition contains dabigatran etexilate free base present with an average particle size in the range of form 1 µm to 100 µm and preferably from 5 µm to 20 µm.

According to an embodiment of the present invention, said pharmaceutical composition of dabigatran etexilate free base may be used for oral, parenteral, intranasal, sublingual, transdermal, transmucosal, ophtalmic, intravenous, pulmonary, intramuscular or rectal administration, and preferably for oral administration.

According to another embodiment of the present invention, said pharmaceutical composition may be formulated as tablets, buccal tablets, sublingual tablets, effervescent tablets, immediate release tablets, modified release tablets, film-coated tablets, orally disintegrating tablets, pills, capsules, caplets, powders, mini tablets, pellets, coated bead systems, granules, microspheres, ion exchange resin systems, steril ocular solutions, aerosols, sprays, drops, ampoules, suppusitories, ocular systems, parenteral systems, creams, gels, ointments, dragees, solutions, elixirs, suspensions or emulsions.

According to another embodiment of the present invention, said composition of dabigatran etexilate free base may comprise one or more pharmaceutically acceptable excipients selected from the group comprising stabilizers, buffering agent, disintegrants, diluents, dispersing agents, binders, lubricants, glidants, plasticizers, preservatives, sweeteners, controlled release agents, flavorings and coloring agents.

Suitable stabilizers may include but not limited to citric acid, fumaric acid, tartaric acid, sodium citrate, sodium benzoate, sodium dihydrogen phosphate, calcium carbonate, magnesium carbonate, arginine, lysine, meglamine, tocopherol, butylhydroxyanisole (BHA), butylhydroxytoluene (BHT), ascorbic acid, gallic acid esters and the mixtures thereof, and preferably, citric acid, fumaric acid, arginine ant mixtures thereof.

In a preferred embodiment of the present invention, said pharmaceutical composition comprises dabigatran etexilate free base as an active agent and at least one stabilizer as a pharmaceutically acceptable excipient. The combination of dabigatran etexilate free base and stabilizer in a unit dosage enhances stability parameter of the dosage form.

Suitable buffering agent may include but not limited to alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide and mixtures thereof, and preferably citric acid, fumaric acid, ascorbic acid, sodium dihydrogen phosphate and mixtures thereof.

In a preferred embodiment of the present invention, said pharmaceutical composition comprises i) dabigatran etexilate free base as an active agent, ii) at least one stabilizer, and iii) at least one buffering agent. Adding buffering agent to adjust pH, provide optimum stability in the pharmaceutical composition of the present invention. Buffering agent minimizes changes in pH of the pharmaceutical composition and provides better solubility and stability of dabigatran etexilate free base which has a solubility strongly dependent to pH.

According to prefered embodiment of the present invention, said pharmaceutical composition contains
a) dabigatran etexilate free base having,
   i. impurity not more than 1% of dabigatran etexilate free base content,
   ii. moisture content not more than 1% of dabigatran etexilate free base content,
   iii. solubility in pure water in the range of 0.5 mg/mL and 3 mg/mL.
b) at least one stabilizer, and
c) at least one buffering agent.

According to prefered embodiment of the present invention, said pharmaceutical composition contains
a) dabigatran etexilate free base having,
   i. impurity not more than 0.4% of dabigatran etexilate free base content,
   ii. moisture content not more than 0.5% of dabigatran etexilate free base content,
   iii. solubility in pure water in the range of 1 mg/mL and 2 mg/mL
b) at least one stabilizer, and
c) at least one buffering agent.

Suitable disintegrants may include but not limited to cross-linked polyvinil pyrrolidone (crospovidone), povidone, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate and mixtures thereof.

Suitable diluents may include but not limited to microcrystalline cellulose, mannitol, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate and mixtures thereof.

Suitable dispersing agents may include but not limited to calcium silicate, magnesium aluminum silicate and mixtures thereof.

Suitable binders may include but not limited to polyvinylpyrrolidone, polyethylene glycol, polyvinyl alcohol, starch, pregelatinized starch, glucose, glucose syrup, natural gums, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, proteins like gelatin, agar, alginate, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide and mixtures thereof.

Suitable lubricants may include but not limited to magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate and mixtures thereof.

Suitable glidants may include but not limited to colloidal silicon dioxide, talc, aluminium silicate, silica and mixtures thereof.

Suitable plasticizers may include but not limited to polyethylene glycols of different molecular weights, propylene glycol and the mixture thereof.

Suitable preservatives may comprise but not limited to methyl paraben and propyl paraben and their salts (such as sodium, potassium), sodium benzoate, citric acid, benzoic acid, butylated hydroxytoluene and butylated hydroxyanisole and mixtures thereof.

Suitable sweeteners may include but not limited to aspartame, potassium acesulfame, sodium saccharinate, neohesperidine dihydrochalcone, sucralose, saccharin, sugars such as sucrose, glucose, lactose, fructose and sugar alcohols such as mannitol, sorbitol, xylitol, erythritol and mixtures thereof.

Suitable flavorings may include but not limited to menthol, peppermint, cinnamon, chocolate, vanillin and fruit essences such as cherry, orange, strawberry, grape, black currant, raspberry, banana, red fruits, wild berries etc. and mixtures thereof.

Suitable coloring agents may include but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow and mixtures thereof.

According to an embodiment of the present invention, said pharmaceutical composition may be formulated as a modified release dosage form comprising at least one controlled release agent as a pharmaceutically acceptable excipient. Said modified release dosage form can be a tablet, capsule, pellets, pellets in capsule, pellets in tablet, mini tablets, suspensions, powders, granules, sachet, caplet or pills.

Suitable controlled release agents of the invention may include, but not limited to, ethyl acrylate- methyl methacylate-trimethylamonioethyl methacrylate copolymer (Eudragit RL;1:2:0.2 and Eudragit;1:2:0.1), methacrylic acid, polyvinil acetate, (collidon SR), methacrylic acid:ethacrylate copolymer (1:1) (Eudragit L100-55) (kollicoat MEA 30DP), cellulose acetate phthalate, ammonium methacrylate copolymers, cellulose acetate trimellitate, hydroxypropyl methylcellulose phthalate, polyvinyl acetate phthalate, hydroxypropyl methylcellulose acetate succinate, hydrogels, carboxyvinyl polymer, sodium alginate, sodium carmellose, calcium carmellose, polyvinyl alcohol, polyvinyl alcohol-polyethylene glycol mixtures, gelatine, starch, polyvinylpyrrolidone, microcrystalline cellulose, collagen, hydroxypropyl methylcellulose, hydroxyethyl cellulose, hydroxypropyl cellulose, ethyl cellulose, carboxymethyl cellulose, carnauba wax, pectin, carbomer, poloxamer, polyachrylamide, aluminium hydroxide, bentonit, laponit, setostearyl alcohol, polyoxyethylen-alkyl ethers, hyaluronic acid, guar gum, cocoa oil, paraffin, hydrogenated vegetable oil, cetyl alcohol, stearyl alcohol, stearic acid, xanthane gum or mixtures thereof.

According to an embodiment of the present invention, pharmaceutical compositions of the present invention may be prepared by conventional technology well known to those skilled in the art such as direct compression, dry granulation, wet granulation and the like. During direct compression, active agent and excipients are mixed, sieved and compressed into dosage forms. During wet granulation, the ingredients are mixed and granulated with a granulation liquid. The granulation process provides agglomerates with a desired homogeneity. The mixture is sieved and optionally mixed with additional excipients. Finally, it is compressed into dosage forms.

## Claims

1. A pharmaceutical composition comprising dabigatran etexilate free base as an active agent and at least one pharmaceutically acceptable excipient.

2. The pharmaceutical composition according to claim 1, wherein dabigatran etexilate free base has an impurity not more than 1% of dabigatran etexilate free base content, which is determined by high performance liquid chromatography (HPLC).

3. The pharmaceutical composition according to claim 1, wherein dabigatran etexilate free base has moisture content not more than 1% of dabigatran etexilate free base content.

4. The pharmaceutical composition according to claim 1, wherein the solubility of dabigatran etexilate free base in pure water has a value between 0.5 mg/mL and 3 mg/mL.

5. The pharmaceutical composition according to any preceeding claims, wherein dabigatran etexilate free base has,
i. impurity not more than 1% of dabigatran etexilate free base content,
ii. moisture content not more than 1% of dabigatran etexilate free base content,
iii. solubility in pure water in the range of 0.5 mg/mL and 3 mg/mL.

6. The pharmaceutical composition according to claim 1, wherein dabigatran etexilate free base has an average particle size in the range of form 1 µm to 100 µm.

7. The pharmaceutical composition according to claim 1, wherein dabigatran etexilate free base is present in an amount ranging between 5 mg and 300 mg.

8. The pharmaceutical composition according to claim 1, which is for oral, parenteral, intranasal, sublingual, transdermal, transmucosal, ophtalmic, intravenous, pulmonary, intramuscular or rectal administration.

9. The pharmaceutical composition according to claim 8, which is for oral administration.

10. The pharmaceutical composition according to claim 1, wherein at least one pharmaceutically acceptable excipient is selected from the group comprising stabilizers, buffering agent, disintegrants, diluents, dispersing agents, binders, lubricants, glidants, plasticizers, preservatives, sweeteners, controlled release agents, flavorings and coloring agents.

11. The pharmaceutical composition according to claim 10, wherein stabilizers is selected from the group comprising citric acid, fumaric acid, tartaric acid, sodium citrate, sodium benzoate, sodium dihydrogen phosphate, calcium carbonate, magnesium carbonate, arginine, lysine, meglamine, tocopherol, butylhydroxyanisole (BHA), butylhydroxytoluene (BHT), ascorbic acid, gallic acid esters and the mixtures thereof.

12. The pharmaceutical composition according to claim 10, wherein buffering agent is selected from alkali metal citrate, citric acid/sodium citrate, potassium hydrogen tartrate, sodium hydrogen tartrate, potassium hydrogen phthalate, sodium hydrogen phthalate, potassium dihydrogen phosphate, disodium hydrogen phosphate, hydrochloric acid/sodium hydroxide and mixtures thereof.

13. The pharmaceutical composition according to any preceeding claims, comprising
i. dabigatran etexilate free base as an active agent,
ii. at least one stabilizer, and
iii. at least one buffering agent.
